# EUROPEAN PATENT SPECIFICATION

(11) **EP 2 797 649 B1**
(45) Date of publication and mention of the grant of the patent: **22.02.2017**
(21) Application number: 12823046.3
(22) Date of filing: 20.12.2012
(51) Int. Cl.: A61M 5/19, A61B 17/00, A61M 5/315

(54) **SYSTEM FOR DELIVERY OF FLUIDS AND USE THEREOF**
SYSTEM ZUR ABGABE VON FLUIDEN UND VERWENDUNG DAVON
SYSTÈME D'ADMINISTRATION DE FLUIDES ET UTILISATION ASSOCIÉE

(30) Priority: 29.12.2011 IL 21727211; 03.01.2012 US 201261582532 P
(43) Date of publication of application: 05.11.2014
(73) Proprietor: Omrix Biopharmaceuticals Ltd., Rehovot 76106 (IL)
(72) Inventor: MERON, Moti, Herzliah 46352 (IL)
(74) Representative: Carpmaels & Ransford LLP
(86) International application number: PCT/IL2012/000395
(87) International publication number: WO 2013/098806

(56) References cited:
- WO-A1-94/03392
- WO-A1-98/10703
- US-A1- 2002 042 591

## Description

### FIELD OF THE INVENTION

Embodiments of the present invention relate to syringe-based system and related methods of use.

### BACKGROUND AND RELATED ART

WO 98/10703 discloses an applicator device for applying a multiple component fluid. The device comprises a plurality of supply containers, each of which is mechanically connected via a fluid control device to an applicator headpiece. The fluid control device is moveable between loading and dispensing configurations.

### SUMMARY

The invention relates to a system for delivery of fluids, the system comprising a syringe having an adjustable length plunger slidable within a respective syringe barrel.

Also, the invention relates to a multi-syringe system where a coupling element causes a plurality of plungers to slide in unison within their respective barrels to simultaneously dispense fluids therefrom. In the system, at least one of the plungers is a variable-length plunger that is selectively engagable to a coupling element.

It is now disclosed a system for delivery of fluids comprising: a. a syringe assembly comprising like-oriented first and second syringes to define a dispensing direction, each syringe having a respective plunger slidable within a respective syringe barrel, the plunger of the second syringe having an adjustable length; and b. a coupling element attached to and mechanically engaged to the plunger of the first syringe so that: i. lengthening or shortening of the adjustable-length plunger of the second syringe respectively causes the adjustable-length plunger of the second syringe to mechanically engage to or disengage from the coupling element; and ii. motion of coupling element in the dispensing direction causes each mechanically engaged plunger to longitudinally slide within its respective syringe barrel to move in unison with the coupling element.

In some embodiments, the coupling element is permanently attached to the plunger of the first syringe.

In some embodiments, the coupling element is integrally formed with and/or glued to the plunger of the first syringe.

In some embodiments, the coupling element is locked to the plunger of the first syringe.

In some embodiments, the coupling element is detachably attached to the plunger of the first syringe.

In some embodiments, the coupling element is clamped to the plunger of the first syringe.

In some embodiments i. the dispensing direction defined by the first and second syringes and ii. a primary direction of contact between respective contact surfaces of the coupling element and of the adjustable-length plunger when mechanically engaged to each other are like-oriented.

In some embodiments, the plunger of second syringe includes a screw mechanism configured to modify a length of the plunger of second syringe.

In some embodiments, the plunger of second syringe includes a screw mechanism configured to modify a length of the plunger of second syringe.

In some embodiments, rotation of a rotatable element around an axis parallel to and/or co-linear with a central axis of second syringe is operative to adjust a length of the adjustable-length plunger of the second syringe to engage to or disengage from the coupling element.

In some embodiments, the plunger of the second syringe comprises: i. an internally threaded sleeve; and ii. externally threaded shaft arranged within the internally threaded sleeve such that rotation of the shaft within the sleeve causes longitudinal motion of the shaft relative to the sleeve, thereby adjusting the length of the plunger of second syringe.

In some embodiments, the syringe assembly further comprises a third syringe that is like-oriented with the first and second syringes, a plunger of the third syringe being mechanically coupled to the coupling element such that motion thereof causes the plunger of the third syringe to longitudinally slide within its respective syringe barrel to move in unison with the coupling element.

In some embodiments, the coupling element is permanently attached to the plunger of the third syringe.

In some embodiments, the coupling element is integrally formed with and/or glued to the plunger of the third syringe.

In some embodiments, the coupling element is locked to the plunger of the third syringe.

In some embodiments, the coupling element is detachably attached to the plunger of the third syringe.

In some embodiments, the coupling element is clamped to the plunger of the third syringe.

In some embodiments, when a central axis of the third syringe substantially equidistant from central axes of the first and the third syringes.

In some embodiments, a cross-sectional area of the barrel of the first syringe and/or a cross-sectional area of the barrel the second syringe is equal to that of the third syringe.

In some embodiments, a cross-sectional area of the barrel of the first syringe and/or a cross-sectional area of the barrel the second syringe different from that of the third syringe.

In some embodiments, the system further comprises a fluid delivery catheter including first and second lumen embedded therein spanning substantially an entirety thereof and configured to respectively receive fluid components discharged from respective barrels of the first and third syringes to define separate channels of fluid component delivery to a tip of lumen.

In some embodiments, the system further comprises a e. a fluid discharge conduit configured to receive fluids discharged from the barrel of the second syringe, an outlet of the barrel of the first syringe being in fluid communication with an exit location of the fluid discharge conduit so that fluids exiting the barrel of the first syringe via fluid discharge conduit mix with fluids exiting the barrel of the second syringe *en route* to a proximal end of the first lumen within the fluid deliver catheter.

In some embodiments, the system further comprises a comprising a check valve configured to regulate flow through the fluid discharge conduit so as to substantially prevent of fluids from the outlet thereof back into barrel of the second syringe.

In some embodiments, a ratio between the longer and shorter lengths of the plunger of the second syringe is at least 1.05 or at least 1.1 or at least 1.15 or at least 1.2 or at least 1.25 or at least 1.3.

In some embodiments, a length difference between the longer and shorter lengths is at least 1 cm.

In some embodiments, a difference between the longer and shorter lengths is at least 5% or at least 10% or at least 15% or at least 20% or at least 25% or at least 30% of an internal length of the barrel of the second syringe.

In some embodiments, the coupling element has a recess dimensioned to match a protruding portion of the second syringe plunger located at a proximal portion thereof.

In some embodiments, a cross-sectional area of barrel of the first syringe is equal to that of the second syringe.

In some embodiments, across-section-area of a barrel of the first syringe differs from that of the second syringe.

In some embodiments, a cross-sectional area of barrel of the first syringe is equal to that of the second syringe.

In some embodiments, the system further comprises: c. a fluid discharge conduit configured to receive fluids discharged from the barrel of the second syringe, an outlet of the barrel of the first syringe being in fluid communication with an exit location of the fluid discharge conduit so that fluids exiting the barrel of the second syringe via fluid discharge conduit mix with fluids exiting the barrel of the first syringe.

In some embodiments, the system further comprises a check valve configured to regulate flow through the fluid discharge conduit so as to substantially prevent of fluids from the outlet thereof back into barrel of the second syringe.

In some embodiments, the system further comprises a fluid delivery catheter including at least one lumen located therein operative to receive the mixture of fluids from the barrels of the first and second syringes.

In some embodiments, the system further comprising: a spike cup including a spike cup conduit therein between a bottom of the spike cup and an interior location within the spike cup, an upper end of the conduit being a sharp end for puncturing a septum of a vial reservoir containing a loadable fluid; a loading port directly or indirectly attached to a given barrel of one of the syringes and configured to receive a lower end of the spike cup conduit so that when engaged thereof fluid flows through the spike cup conduit into the given barrel so as to load the given barrel.

In some embodiments, the loading port is rotatable between open and closed configurations such that only when in the open configuration is the loading port is open to receive fluid therein.

It is now disclosed fibrinogen and thrombin for use in a method of therapy, wherein said fibrinogen and thrombin are administered by a method for delivery of fluids comprising: a. providing a syringe assembly comprising: i. like-oriented first and second syringes to define a dispensing direction, each syringe having a respective plunger slidable within a respective syringe barrel, the plunger of the second syringe having an adjustable length; and ii. a coupling element attached to and mechanically engaged to the plunger of the first syringe b. lengthening or shortening of the adjustable-length plunger of the second syringe so as to cause the adjustable-length plunger of the second syringe to mechanically engage to or disengage from the coupling element; and c. moving coupling element in the dispensing direction so as to cause each mechanically engaged plunger to longitudinally slide within its respective syringe barrel to move in unison with the coupling element and thereby causing fluids comprising fibrinogen and thrombin to be simultaneously expelled from respective barrels.

### BRIEF DESCRIPTION OF THE DRAWINGS

FIGS. 1A-1B, 2A-2B illustrate a multi-syringe system including a selectively-engagable syringe plunger that is decoupled from a coupling element.
FIGS. 3A-3B, 4A-4B illustrate the same system when selectively-engagable syringe plunger that is coupled to the coupling element.
FIG. 5 illustrates a method of operating the same system.
FIGS. 6A-6B illustrate a first variable-length plunger.
FIGS. 7A illustrate a proximal end of a plunger when disengaged from the coupling element.
FIGS. 7B illustrate a proximal end of a plunger when engaged to the coupling element.
FIG. 8 illustrates a technique for loading the system.

### DETAILED DESCRIPTION OF EMBODIMENTS

Embodiments of the present invention relate to a multi-syringe system/device providing multiple operating modes. When the device is in a first operating mode, *N (N* is a positive integer equal to two or more - i.e. *N* ≥ 2) like-oriented syringes are coupled together so that motion of a so-called coupling element in a dispensing direction causes multiple plungers to move in unison within each of their respective barrels so that fluid components are simultaneously dispensed from each barrel of the *N* syringes. The coupling element connects the multiple plungers to each other so that plungers within all *N* barrels slide together in unison.

When in a second operating mode, motion of the coupling element in the dispensing direction causes plunger(s) to move in unison within only *M* barrels where *M* is a positive integer less than *N* (i.e. 1 ≤ *M* < *N*). Thus, in the second operating mode, motion of the coupling element causes fluid components to be dispensed from respective barrel(s) of *M* syringe(s).

In one non-limiting example, a single multi-syringe device may be operated, at the user's discretion either in an *N*-component mode to simultaneously administer *N* components or in *M*-component mode to simultaneously administer *M* components.

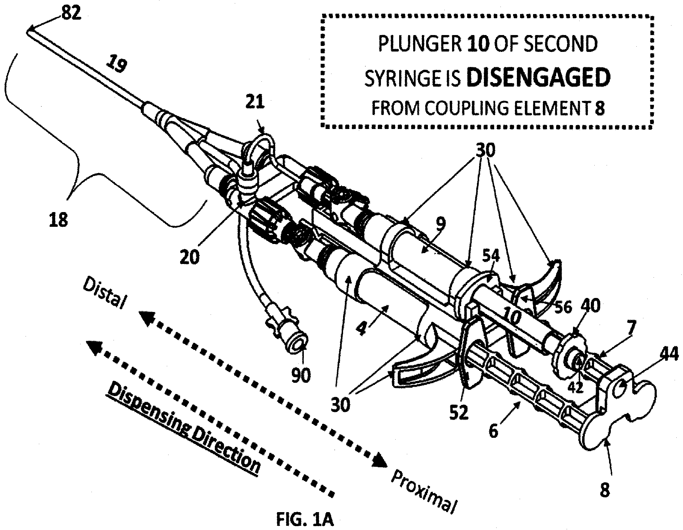

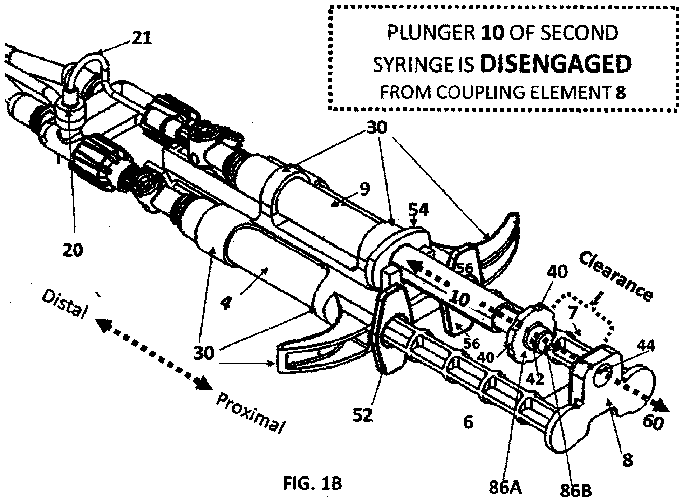

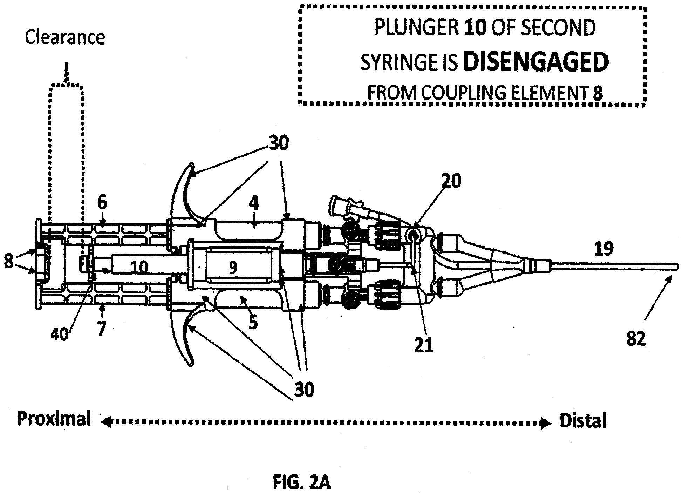

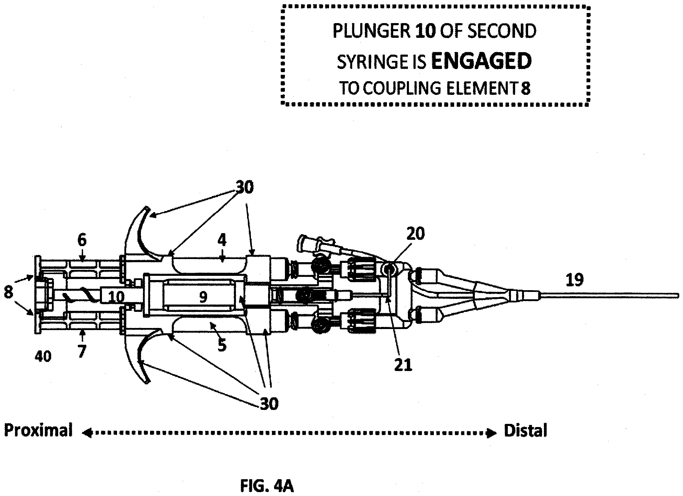

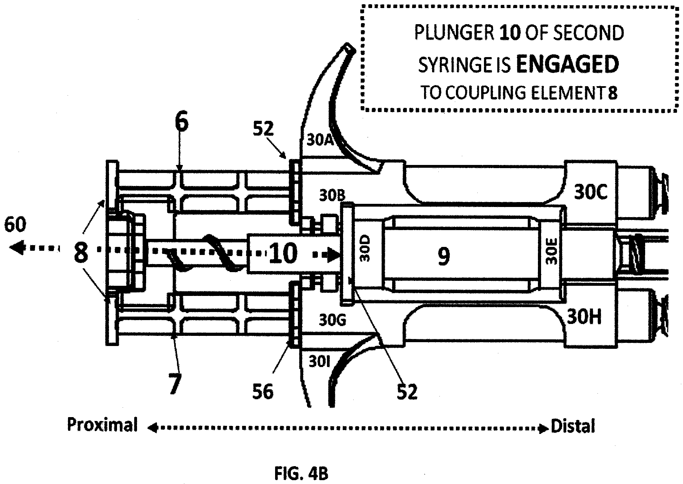

FIGS. 1-4 illustrate a specific multi-plunger device where *N*=3 and *M*=2 including three like-oriented syringes. In the non-limiting example of FIGS. 1-4, the multi-syringe device includes three syringes, two of which have plungers **6, 7** that are permanently attached to coupling element 8. In FIG. 1A, first and second syringes respectively having barrels **4,9** are visible. In FIG. 2A, a barrel **5** of a third syringe within which a third plunger **7** slides is visible.

Because plungers **6,7** of the first and third syringes are permanently attached to coupling element **8,** in general, motion of coupling element **8** in a dispensing direction causes plungers **6, 7** to move in unison with each other and with coupling element **8** respectively within barrels **4,5** to simultaneously dispense fluid therefrom.

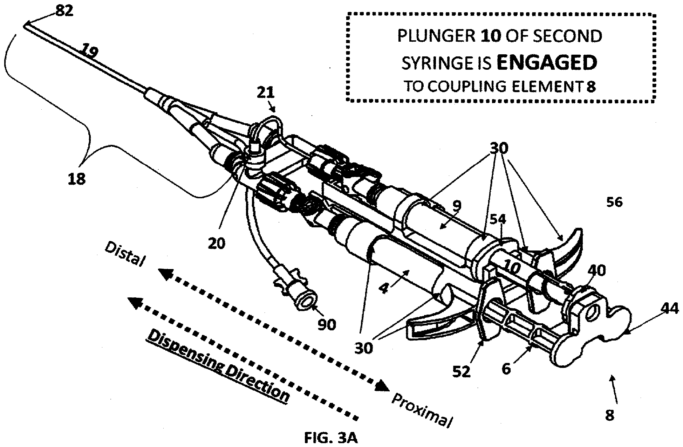

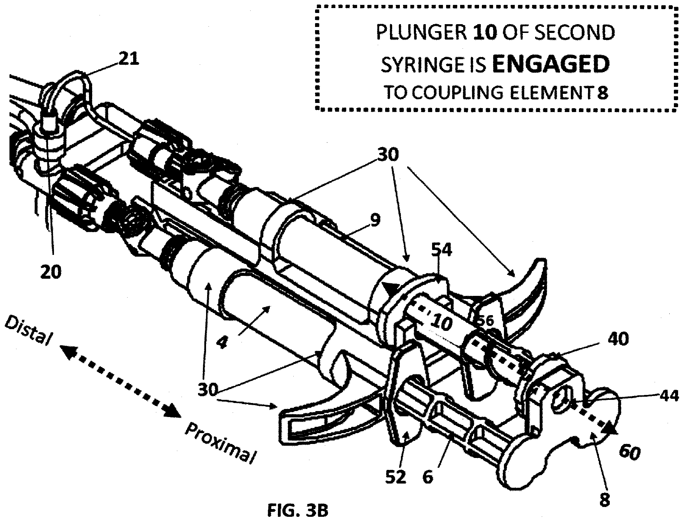

One salient feature of the multi-syringe device is that plunger **10** (i.e. arranged within the barrel **9** of the second syringe) is selectively couplable/engable to coupling element **8.** When plunger **10** is coupled/engaged as in FIGS. 3-4, motion of coupling element **8** in a dispensing direction causes selectively couplable/engable plunger **10** to move in unison with plungers **6, 7.** Simultaneous motion of plungers **6, 7,10** within respective barrels **4, 5, 9** causes fluid to be simultaneously expelled or dispensed from all three syringe barrels **4,5,9.**

As will be discussed below, in some embodiments, plunger **10** is a variable length plunger - lengthening of variable-length plunger **10** is operative to bring a proximal end thereof into contact with coupling element **8** to mechanically engage thereto.

The like-oriented syringes are all substantially parallel to each other so as to define a dispensing direction - as illustrated in figure 1A, the dispensing direction is co-oriented with a 'proximal-distal vector' orientation.

FIGS. 1-2 illustrate the multi-syringe device when in 'two-component' operating mode. When in two-component mode, coupling element **8** is disengaged from selectively couplable plunger **10,** and coupled to permanently-attached plungers **6, 7.** When in two-component mode, a single stroke of coupling element **8** causes plungers **6, 7** to simultaneously slide within respective syringe barrels **4,5** so that fluids are simultaneously expelled therefrom. In one use case, it is possible to simultaneously dispense a fibrinogen component from barrel **4** and a thrombin component from barrel **5**- for example, so that upon separately exiting barrels **4,5** each component separately enters into respective lumens within catheter **19** and exits a distal end **82** thereof. In this use case, the components (i.e. fibrinogen and the thrombin) may only mix with each other after exiting catheter **19.**

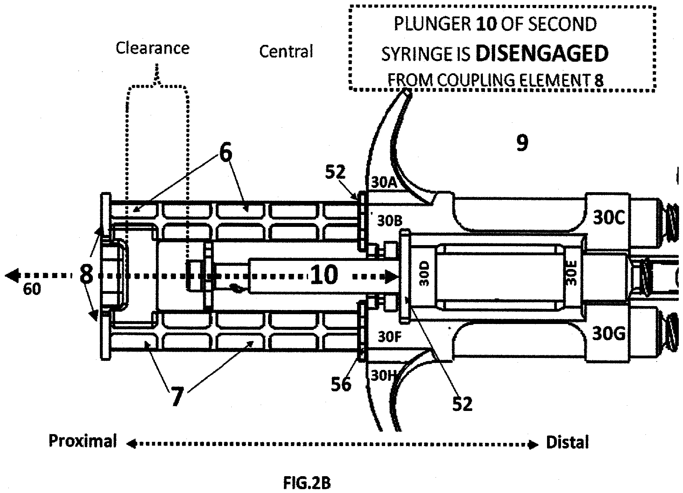

Also illustrated in FIGS. 1B, 2A and 2B (i.e. relating to the 'two-component' operating mode) is a 'clearance' between (i) a proximal end of plunger **10** and (ii) coupling element **8.** When the multi-syringe device is in the 'two-component' operating mode of FIGS. 1-2, motion along the 'proximal-distal' axis or 'dispensing' axis of coupling element **8** causes in-tandem motion of the permanently attached plungers **6, 7** without setting plunger **10** in motion - fluid is thus expelled from barrels **4,5** and not from barrel **9.**

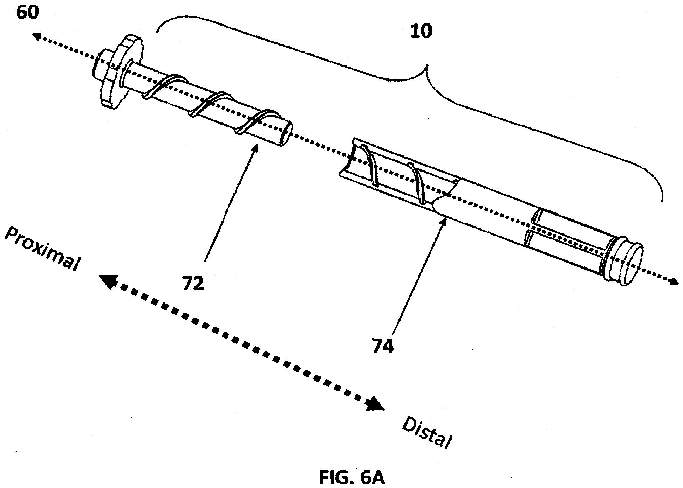

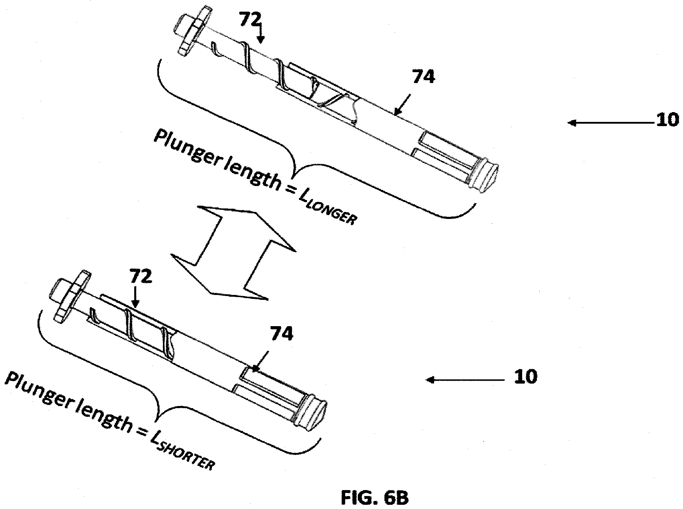

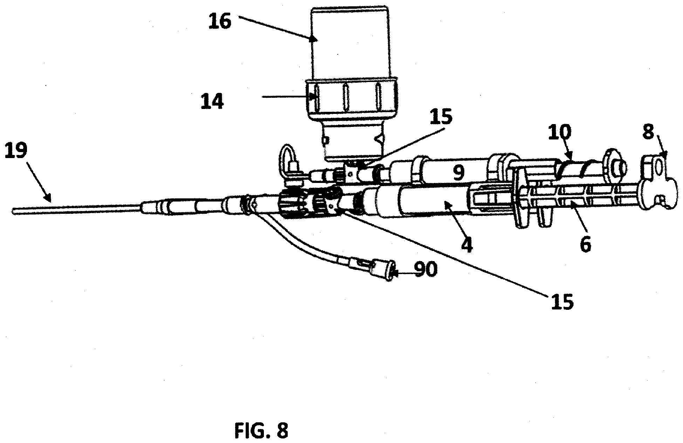

FIG. 3-4 describe the multi-syringe device in the 'three-component' operating mode. In the non-limiting examples of FIGS. 1-5, plunger **10** of multi-syringe device is a so-called 'variable length plunger' - when the plunger length is 'shorter' multi-syringe device is in two-component mode, discussed above. In order to transition from two-component mode to three-component mode, it is possible to increase a length of variable-length plunger - non-limiting examples of accomplishing this are discussed below with reference to FIGS. 6 and 8.

When in three-component mode, coupling element **8** is in contact with a proximal location on plunger **10** so that plunger **10** may be said to be 'coupled to' coupling element **8.** In this mode, motion of coupling element **8** in the dispensing direction causes sliding motion of three plungers **6, 7,10** within respective barrels **4, 5, 9** so that fluid components are respectively and simultaneously expelled from each of the three barrels **4,5,** and **9.** In this mode, coupling element **8** pushes upon plunger **10** to force plunger **10** to slide within its barrel **9.**

In one non-limiting use case, (i) when the multi-syringe device is in two component mode, only fibrinogen and thrombin components are administered to a patient and (ii) when the multi-syringe device is in three component mode, it is possible to simultaneously administer fibrinogen (i.e. from barrel **4**), thrombin (i.e. from barrel **5**) and a supplement (e.g. antibiotics, anti-inflammatory agents, chemotherapy agents, growth factors, anti-cancer drugs analgesics, proteins, hormones, antioxidants and the like) from barrel **9**. The same multi-syringe device may thus be employed, at the user's discretion, in either two-component mode or three-component mode, allowing a practitioner (e.g. a surgeon) to utilize the same multi-syringe device for both purposes.

In one example, the fibrinogen component and thrombin component can be administered simultaneously and the supplement may be administered at any time during the administration of the two components, at the user's discretion. Thus, at one time point during the administration, the user may decide to administer the supplement along with the fibrinogen component and thrombin component whereas at another time point, the user may decide to administer only the two components without the supplement, meaning that some portion of the fibrin sealant will include the supplement while other portions will only consist fibrin sealant.

The step of engagement and disengagement of plunger **10** from coupling element **8** can be carried out alternately during the administration of the liquid components. For example, the administration can be initiated wherein plunger **10** is disengaged from coupling element **8** and multi-syringe device is in 'two-component mode. In a second step, plunger **10** can be engaged with coupling element **8** followed by a third step of disengaging plunger **10** from coupling element **8.**

The administration can be carried out by injection e.g. when a needle is installed on the dispensing end of the device, by dripping, or by spraying (e.g. when a gas inlet **90** e.g. a pressurized gas is added so that an inlet gas stream mixes with fluid dispensed from the syringe barrel(s)). The administration can also be carried out by casting the components into a mold.

One feature of the device illustrated in FIGS. 1-4 is that the diameters of barrels **4, 5, 9** are equal to each other - thus, movement of coupling element **8** in a dispensing direction causes equal volumes of fluid to be expelled respectively from each barrel **4, 5, 9.** This is not a limitation. In other embodiments, the diameters of two or more of barrels **4, 5, 9** may differ from each other. It is possible to manufacture the device to control a ratio between diameters of two or more barrels according to a desired ratio between the fluid components to be dispensed therefrom.

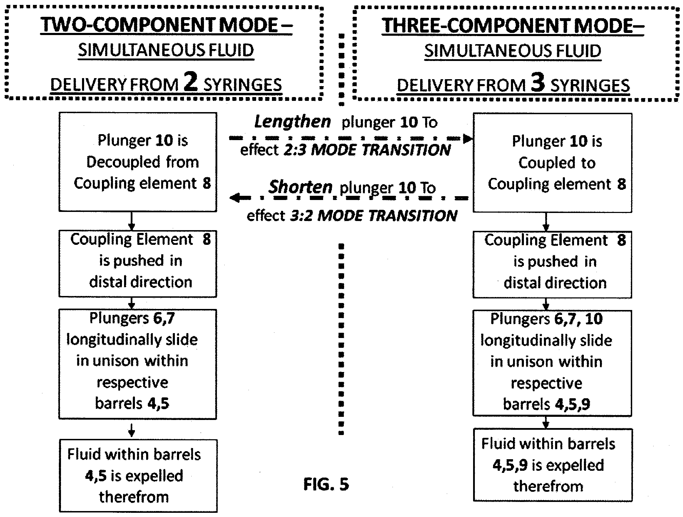

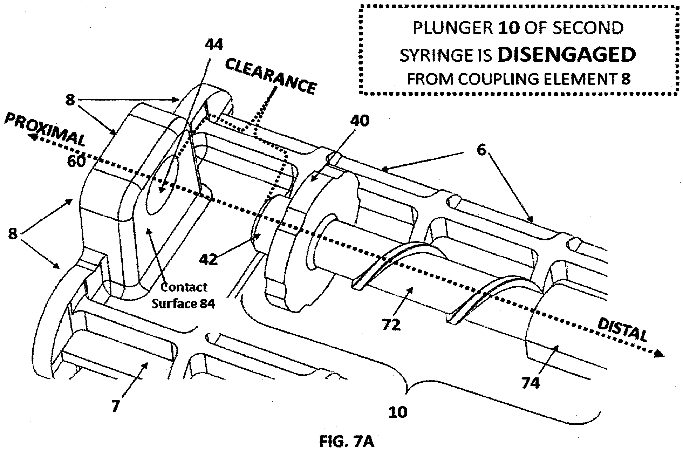

FIG. 5 is a flow chart of a technique of operating the multi-syringe device. As illustrated in FIG.5, when plunger **10** is lengthened (i.e. sufficiently so that a proximal portion of the plunger **10** contacts a 'distal-facing' surface (see **84** of FIG. 7A) of coupling element **8**), the multi-syringe device transitions from the 'two-component mode' to the 'three-component' mode. This is referred to as a 2:3 mode transition in FIG. 5. When the multi-syringe device is in 'three-component mode' and plunger **10** is shortened (i.e. to eliminate contact between coupling element **8** and plunger **10**), the multi-syringe device transitions from 'three-component mode' to 'two-component mode'- this is referred to as 3:2 mode transition in FIG. 5. As indicated in the figure, when in two-component mode, movement of coupling element **8** in a dispensing direction (i.e. substantially parallel to a 'proximal-distal' direction) causes two plungers **6, 7** to slide in unison within their respective barrels **4,5** and fluid to be expelled therefrom. When in three-component mode, movement of coupling element **8** in a dispensing direction additionally causes plunger **10** to slide in unison with plungers **6, 7** so that fluid is expelled simultaneously from three syringe barrels **4,5, 9.**

Typically, coupling element **8** imparts momentum about plunger **10** by pushing plunger **10** without relying on friction or any other additional mechanism. The orientation of a 'contact' surface **84** of coupling element **8** in contact with plunger **10** is defined by its local normal. As shown in FIG. 7A, this contact surface faces the distal direction. The orientation of the 'contact' surface (see **86A** and/or **86B** of FIG. 1B) of plunger **10** in contact with coupling element **8** is in the opposite direction - i.e. facing the proximal direction.

Thus, a 'contact direction' from the coupling element to the adjustable-length plunger is substantially in the dispensing direction along the proximal-distal axis of the multi-syringe device. It may thus be said that the direction of contact from the coupling element **8** to plunger **10** is like-oriented with a dispensing direction. This allows coupling element **8** to push plunger **10** by applying a force along the contact direction, thereby causing plunger **10** to slide in a dispensing direction to expel or dispense fluid from a barrel **9** within which plunger **10** slides.

For the present disclosure, the term 'fluid' is defined broadly and may refer to any flowable matter including but not limited to liquids and flowable gels.

As discussed above, in some embodiments, plunger **10** may be a variable-length plunger. One example of a variable length plunger is illustrated in FIGS. 6A-6B. In this example, variable length plunger **10** includes an externally-threaded shaft **72** rotatable within an internally threadable sleeve **74** (this feature is shown in a broken out section in order to see its internal structure). Rotation of the shaft **72** around a central axis **60** of plunger **10** causes longitudinal motion of shaft **72** within sleeve **74** to modify a position of shaft **72** relative to sleeve **74,** and lengthen or shorten variable-length plunger. The direction of rotation in FIG. 6 is just an example and other rotation mechanisms for lengthening or shortening a length of plunger **10** (for example, which require rotating around a rotation axis having a different orientation) are contemplated.

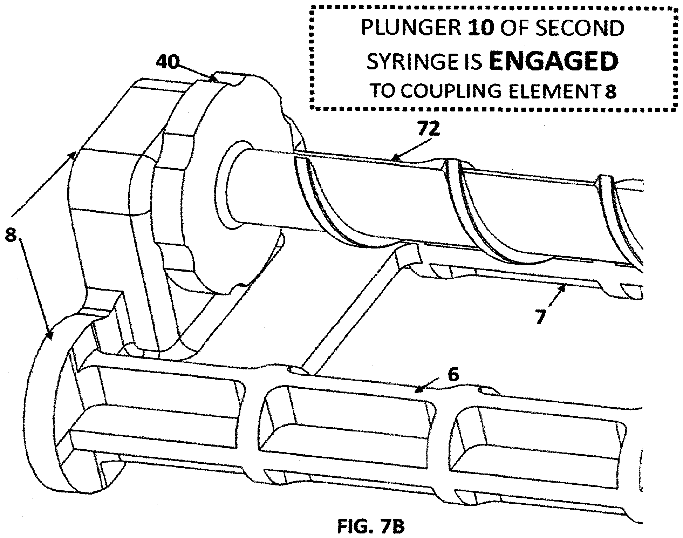

FIGS. 7A-7B illustrate a proximal region of the variable-length plunger of FIGS. 6A-6B in proximity with coupling element **8** at a time plunger **10** is disengaged from coupling element **8.** As illustrated in FIG. 7A, there is a clearance between (i) a proximal portion of plunger **10;** and (ii) a distal portion of coupling element **8.** In FIG. 7B, after plunger **10** is lengthened, no such clearance exists - instead, there is contact between a distal-facing surface **84** of coupling element **8** and plunger **10.** In the non-limiting example of FIG. 7, variable-length plunger **10** includes a shoulder **40** portion and a proximally-protruding element **42** protruding from shoulder **40** which is dimensioned to fit within recess **44** of coupling element **8.**

Also illustrated in FIGS. 7A-7B are plungers **6, 7** which are permanently attached to coupling element **8.** One example of 'permanently attached' plungers **6, 7** are plungers glued to coupling element **8.** In another example, plungers **6, 7** are integrally formed with coupling element **8** - this may reduce the number of parts required to manufacture the multi-syringe device. It is noted that the 'permanently attached' feature is not a requirement - in another example, plungers **6, 7** are detachably attached to coupling element **8** - for example, by a snap or any other mechanism.

Additional elements illustrated in FIGS. 1-4 are now discussed. Multi-syringe device includes manifold **18** through which fluids delivered from syringe barrels **4,5,9** pass to exit the device. In some embodiments, manifold **18** includes catheter **19** having multiple lumen to provide separate flow 'channels'. Fluids components (e.g. fibrinogen and thrombin) expelled from syringe barrels **4,5** subsequently and separately flow through catheter **19** and exit distal end **82** thereof so as to be dispensed separately - this may be useful for 'surgical glue' applications.

In the example of FIGS. 1-4, fluids expelled from barrel **9** may be introduced into a flow path between an outlet of syringe barrel **4** and a proximal end of catheter **19** so as to mix with each other before entering "fluid delivery" catheter **19.** For example, fluid expelled from barrel **9** flows through fluid discharge conduit **21** and one-way valve **20** or 'check valve' into a location along the aforementioned flow path. One-way valve **20** prevents "back-flow" of a fluid component exiting barrel **4** from flowing into barrel **9.**

Also visible in FIGS. 1-4 are the following elements: (i) attachment/holding element or brace **30** which holds the first, second and third syringes together in the same general orientation; (ii) shoulders **52, 54, 56** of the first, second and third syringes; and (iii) central axis **60** of the barrel **9** of the second syringe and/or the plunger **10** therein. Attachment/holding element **30** includes various portions **30A-30H** labeled in FIG. 2B.

As noted above, FIGS. 6A-6B describe a first mechanism for varying a plunger length **10.** In some embodiments, the 'rotation/screw' mechanism provides an ease-of-use and may require fewer parts when manufacturing the multi-syringe device. The mechanisms for lengthening or shortening the plunger described herein is an example - the skilled artisan will recognize that various mechanisms may be employed to accomplish this task.

In the examples of FIGS. 1-7 one or more of the plungers is reversibly or 'selectively' engagable to coupling element. The mechanism for providing this feature includes a variable length plunger.

FIG. 8 relate to techniques for loading fluids into the multi-syringe device in accordance with some embodiments.

Prior to use, the barrels **4,5,9** are loaded with the liquid/fluid components. Loading of the barrels can be carried out by installing a spike cup **14** on a fluid control device/loading port **15,** and placing a vial/reservoir **16** within the spike cup **14.** The spike cup may comprise a protruding needle preferably adapted to puncture the vial's septum. The vial in the spike cup is punctured by the protruding needle, enabling liquid flow from the vial and into the barrel via the needle by drawing the plunger. The plunger is pulled in the opposite direction to the dispensing direction, resulting in drawing of the fluid component from the vial **16** into the barrel. In one example, plungers **6** and **7** (which are connected with each other, e.g. via part **8**) are pulled simultaneously leading to loading of barrels **4** and **5.** Typically, plunger **10** is pulled separately leading to loading of barrel **9.**

Fig. 8 shows the upper part of the fluid control device corresponding to each syringe, revealing the connection interface having a designated structure for connecting with the spike cup. A spike cup **14** and a vial **16** connected to the fluid control device **15** of the second syringe is shown in Fig. 10.

Following loading of the barrels with the liquid components, the spike cup **14** and the vial **16** are removed by rotating the spike cup, thus allowing, in a subsequent step, dispensing of the liquid components from the barrels.

The structure and function of the fluid control device and its use for loading the barrels of the device with the fluid components as well as for dispensing the fluid components are explained in WO98/10703.

In the description and claims of the present application, each of the verbs, "comprise" "include" and "have", and conjugates thereof, are used to indicate that the object or objects of the verb are not necessarily a complete listing of members, components, elements or parts of the subject or subjects of the verb.

The present invention has been described using detailed descriptions of embodiments thereof that are provided by way of example and are not intended to limit the scope of the invention. The described embodiments comprise different features, not all of which are required in all embodiments of the invention. Some embodiments of the present invention utilize only some of the features or possible combinations of the features. Variations of embodiments of the present invention that are described and embodiments of the present invention comprising different combinations of features noted in the described embodiments will occur to persons of the art.

## Claims

1. A system for delivery of fluids, the system comprising:
a. a syringe assembly comprising like-oriented first and second syringes to define a dispensing direction, each syringe having a respective plunger **6,10** slidable within a respective syringe barrel **4,9, characterised in that** the plunger **10** of the second syringe has an adjustable length;
and further **characterised in that** the system comprises:
b. a coupling element **8** attached to and mechanically engaged to the plunger **6** of the first syringe so that:
i. lengthening or shortening of the adjustable-length plunger **10** of the second syringe respectively causes the adjustable-length plunger **10** of the second syringe to mechanically engage to or disengage from the coupling element **8;** and
ii. motion of the coupling element **8** in the dispensing direction causes each mechanically engaged plunger to longitudinally slide within its respective syringe barrel **4,9** to move in unison with the coupling element **8.**

2. The fluid delivery system of claim 1 wherein the coupling element **8** is permanently attached to the plunger **6** of the first syringe; or wherein the coupling element **8** is integrally formed with and/or glued to the plunger **6** of the first syringe; or wherein the coupling element **8** is locked to the plunger **6** of the first syringe; or wherein the coupling element **8** is detachably attached to the plunger **6** of the first syringe; or wherein the coupling element **8** is clamped to the plunger **6** of the first syringe.

3. The fluid delivery system of claim 1 or claim 2 wherein:
i. the dispensing direction defined by the first and second syringes and
ii. a primary direction of contact between respective contact surfaces **86,84** of the coupling element **8** and of the adjustable-length plunger **10** when mechanically engaged to each other, are like-oriented.

4. The fluid delivery system of any preceding claim wherein the plunger **10** of second syringe comprises a screw mechanism configured to modify a length of the plunger **10** of the second syringe, optionally wherein the plunger **10** of the second syringe comprises:
i. an internally **74** threaded sleeve; and
ii. externally threaded shaft **72** arranged within the internally threaded sleeve such that rotation of the shaft within the sleeve **74** causes longitudinal motion of the shaft relative to the sleeve, thereby adjusting the length of the plunger **10** of second syringe.

5. The fluid delivery system of any preceding claim wherein rotation of a rotatable element around an axis parallel to and/or co-linear with a central axis of second syringe is operative to adjust a length of the adjustable-length plunger **10** of the second syringe to engage to or disengage from the coupling element **8.**

6. The fluid delivery system of any preceding claim wherein the syringe assembly further comprises a third syringe that is like-oriented with the first and second syringes, a plunger 7 of the third syringe being mechanically coupled to the coupling element **8** such that motion thereof causes the plunger 7 of the third syringe to longitudinally slide within its respective syringe barrel **5** to move in unison with the coupling element **8.**

7. The fluid delivery system of claim 6 wherein the coupling element **8** is permanently attached to the plunger **7** of the third syringe; or wherein the coupling element **8** is integrally formed with and/or glued to the plunger **7** of the third syringe; or wherein the coupling element **8** is locked to the plunger **7** of the third syringe or wherein the coupling element **8** is detachably attached to the plunger **7** of the third syringe; or wherein the coupling element **8** is clamped to the plunger **7** of the third syringe.

8. The fluid delivery system of claim 6 or claim 7 wherein a central axis **60** of the second syringe is substantially equidistant from central axes of the first and the third syringes.

9. The fluid delivery system of any of claims 6-8 wherein a cross-sectional area of the barrel **4** of the first syringe and/or a cross-sectional area of the barrel **9** of the second syringe is equal to a cross sectional area of the barrel **5** of the third syringe.

10. The fluid delivery system of any of claims 6-8 wherein a cross-sectional area of the barrel **4** of the first syringe and/or a cross-sectional area of the barrel **9** of the second syringe is different from a cross sectional area of the barrel **5** of the third syringe.

11. The fluid delivery system of any of claims 6-10 further comprising a fluid delivery catheter **19** comprising a first and second lumen embedded therein spanning substantially an entirety thereof and configured to respectively receive fluid components discharged from respective barrels **4,5** of the first and third syringes to define separate channels of fluid component delivery to a tip **82** of the catheter **19.**

12. The fluid delivery system of any preceding claim wherein a ratio between the longer and shorter lengths of the plunger 10 of the second syringe is at least 1.2; and/or wherein a length difference between the longer and shorter lengths of the plunger 10 of the second syringe is at least 1 cm; and/or wherein a difference between the longer and shorter lengths of the plunger 10 of the second syringe is at least 15 % of an internal length of the barrel of the second syringe.

13. The fluid delivery system of any preceding claim wherein the coupling element **8** has a recess **44** dimensioned to match a protruding portion **42** of the second syringe plunger **10** located at a proximal portion thereof.

14. The fluid delivery system of any preceding claim wherein a cross-sectional area of the barrel **4** of the first syringe is equal to a cross-sectional area of the barrel **9** of the second syringe.

15. The fluid delivery system of any preceding claim wherein a cross-section-area of a barrel **4** of the first syringe differs from a cross-sectional area of the barrel **9** of the second syringe.

16. The fluid delivery system of any preceding claim further comprising:
c. a fluid discharge conduit **21** configured to receive fluids discharged from the barrel **9** of the second syringe, an outlet of the barrel **4** of the first syringe being in fluid communication with an exit location of the fluid discharge conduit **21** so that fluids exiting the barrel **9** of the second syringe via fluid discharge conduit **21** mix with fluids exiting the barrel **4** of the first syringe; and optionally further comprising a check valve **20** configured to regulate flow through the fluid discharge conduit **21** so as to substantially prevent of fluids from the outlet thereof back into barrel **9** of the second syringe.

17. The fluid delivery system of claim 16 further comprising a fluid delivery catheter **19** comprising at least one lumen located therein operative to receive the mixture of fluids from the barrels **4,9** of the first and second syringes.

18. The fluid delivery device of any preceding claim further comprising:
a spike cup **14** including a spike cup conduit therein between a bottom of the spike cup and an interior location within the spike cup, an upper end of the conduit being a sharp end for puncturing a septum of a vial reservoir **16** containing a loadable fluid;
a loading port **15** directly or indirectly attached to a given barrel of one of the syringes and configured to receive a lower end of the spike cup conduit so that when engaged thereof fluid flows through the spike cup conduit into the given barrel so as to load the given barrel; optionally wherein the loading port is rotatable to open and close configurations such that only when in the open configuration the loading port is open to receive fluid therein.

19. Fibrinogen and thrombin for use in a method of therapy, wherein said fibrinogen and thrombin are administered by a method for delivery of fluids comprising:
providing a syringe assembly comprising:
i. like-oriented first and second syringes to define a dispensing direction, each syringe having a respective plunger **6,10** slidable within a respective syringe barrel **4,9,** the plunger **10** of the second syringe having an adjustable length; and
ii. a coupling element **8** attached to and mechanically engaged to the plunger **6** of the first syringe;
lengthening or shortening of the adjustable-length plunger **10** of the second syringe so as to cause the adjustable-length plunger **10** of the second syringe to mechanically engage to or disengage from the coupling element 8; and
moving coupling element **8** in the dispensing direction so as to cause each engaged plunger to longitudinally slide within its respective syringe barrel **4,9** to move in unison with the coupling element **8** and thereby causing fluids comprising fibrinogen and thrombin to be simultaneously expelled from respective barrels.

## Patentansprüche

1. System zur Abgabe von Fluiden, umfassend:
a. eine Spritzenanordnung, die eine erste und eine zweite Spritze umfasst, die gleich ausgerichtet sind, um eine Verabreichungsrichtung zu definieren, wobei jede Spritze einen jeweiligen Kolben 6, 10 hat, der in einem jeweiligen Spritzenzylinder 4, 9 verschiebbar ist, **dadurch gekennzeichnet, dass** der Kolben 10 der zweiten Spritze eine verstellbare Länge hat,
und ferner **dadurch gekennzeichnet, dass** das System
b. ein Koppelelement 8 umfasst, das an dem Kolben 6 der ersten Spritze angebracht ist und damit mechanisch in Eingriff steht, so dass
i. durch Verlängern oder Verkürzen des Kolbens 10 mit verstellbarer Länge der zweiten Spritze veranlasst wird, dass der Kolben 10 mit verstellbarer Länge der zweiten Spritze das Koppelelement 8 mechanisch in Eingriff nimmt bzw. daraus ausrückt, und
ii. durch eine Bewegung des Koppelelements 8 in der Verabreichungsrichtung veranlasst wird, dass jeder mechanisch in Eingriff stehende Kolben in Längsrichtung in seinem jeweiligen Spritzenzylinder 4, 9 gleitet, um sich übereinstimmend mit dem Koppelelement 8 zu bewegen.

2. Fluidabgabesystem nach Anspruch 1, wobei das Koppelelement 8 permanent am Kolben 6 der ersten Spritze angebracht ist oder wobei das Koppelelement 8 mit dem Kolben 6 der ersten Spritze integral ausgebildet und/oder daran angeklebt ist oder wobei das Koppelelement 8 am Kolben 6 der ersten Spritze verriegelt ist oder wobei das Koppelelement 8 lösbar am Kolben 6 der ersten Spritze angebracht ist oder wobei das Koppelelement 8 an den Kolben 6 der ersten Spritze angeklammert ist.

3. Fluidabgabesystem nach Anspruch 1 oder Anspruch 2, wobei
i. die von der ersten und der zweiten Spritze definierte Verabreichungsrichtung und
ii. eine primäre Richtung des Kontakts zwischen jeweiligen Kontaktflächen 86, 84 des Koppelelements 8 und des Kolbens 10 mit verstellbarer Länge, wenn sie mechanisch miteinander in Eingriff stehen, gleich ausgerichtet sind.

4. Fluidabgabesystem nach einem der vorhergehenden Ansprüche, wobei der Kolben 10 der zweiten Spritze einen Schraubmechanismus umfasst, der dazu konfiguriert ist, eine Länge des Kolbens 10 der zweiten Spritze zu modifizieren, wahlweise wobei der Kolben 10 der zweiten Spritze Folgendes umfasst:
i. eine Hülse 74 mit einem Innengewinde und
ii. eine Welle 72 mit einem Außengewinde, die in der Hülse mit dem Innengewinde angeordnet ist, so dass eine Drehung der Welle in der Hülse 74 eine Längsbewegung der Welle bezüglich der Hülse veranlasst, wodurch die Länge des Kolbens 10 der zweiten Spritze verstellt wird.

5. Fluidabgabesystem nach einem der vorhergehenden Ansprüche, wobei eine Drehung eines drehbaren Elements um eine parallel zu und/oder kollinear mit einer mittleren Achse der zweiten Spritze verlaufende Achse dahingehend wirkt, dass eine Länge des Kolbens 10 mit verstellbarer Länge der zweiten Spritze verstellt wird, um das Koppelelement 8 in Eingriff zu nehmen oder daraus auszurücken.

6. Fluidabgabesystem nach einem der vorhergehenden Ansprüche, wobei die Spritzenanordnung ferner eine dritte Spritze umfasst, die mit der ersten und der zweiten Spritze gleich ausgerichtet ist, wobei ein Kolben 7 der dritten Spritze mechanisch an das Koppelelement 8 gekoppelt ist, so dass durch eine Bewegung davon veranlasst wird, dass der Kolben 7 der dritten Spritze in Längsrichtung in seinem jeweiligen Spritzenzylinder 5 gleitet, um sich übereinstimmend mit dem Koppelelement 8 zu bewegen.

7. Fluidabgabesystem nach Anspruch 6, wobei das Koppelelement 8 permanent am Kolben 7 der dritten Spritze angebracht ist oder wobei das Koppelelement 8 mit dem Kolben 7 der dritten Spritze integral ausgebildet und/oder daran angeklebt ist oder wobei das Koppelelement 8 am Kolben 7 der dritten Spritze verriegelt ist oder wobei das Koppelelement 8 lösbar am Kolben 7 der dritten Spritze angebracht ist oder wobei das Koppelelement 8 an den Kolben 7 der dritten Spritze angeklammert ist.

8. Fluidabgabesystem nach Anspruch 6 oder Anspruch 7, wobei eine mittlere Achse 60 der zweiten Spritze im Wesentlichen gleich weit von mittleren Achsen der ersten und der dritten Spritze entfernt ist.

9. Fluidabgabesystem nach einem der Ansprüche 6 - 8, wobei eine Querschnittsfläche des Zylinders 4 der ersten Spritze und/oder eine Querschnittsfläche des Zylinders 9 der zweiten Spritze gleich einer Querschnittsfläche des Zylinders 5 der dritten Spritze sind.

10. Fluidabgabesystem nach einem der Ansprüche 6 - 8, wobei eine Querschnittsfläche des Zylinders 4 der ersten Spritze und/oder eine Querschnittsfläche des Zylinders 9 der zweiten Spritze von einer Querschnittsfläche des Zylinders 5 der dritten Spritze verschieden sind.

11. Fluidabgabesystem nach einem der Ansprüche 6 - 10, ferner umfassend einen Fluidabgabekatheter 19, der ein erstes und ein zweites Lumen umfasst, die darin eingebettet sind, im Wesentlichen eine Gesamtheit davon überspannen und dazu konfiguriert sind, jeweils von jeweiligen Zylindern 4, 5 der ersten und der dritten Spritze ausgetragene Fluidkomponenten aufzunehmen, um getrennte Kanäle zur Abgabe von Fluidkomponenten an eine Spitze 82 des Katheters 19 zu definieren.

12. Fluidabgabesystem nach einem der vorhergehenden Ansprüche, wobei ein Verhältnis zwischen den längeren und kürzeren Längen des Kolbens 10 der zweiten Spritze mindestens 1,2 beträgt und/oder wobei ein Längenunterschied zwischen den längeren und kürzeren Längen des Kolbens 10 der zweiten Spritze mindestens 1 cm beträgt und/oder wobei ein Unterschied zwischen den längeren und kürzeren Längen des Kolbens 10 der zweiten Spritze mindestens 15% einer inneren Länge des Zylinders der zweiten Spritze beträgt.

13. Fluidabgabesystem nach einem der vorhergehenden Ansprüche, wobei das Koppelelement 8 eine Aussparung 44 hat, die so bemessen ist, dass sie mit einem vorragenden Abschnitt 42 des Kolbens 10 der zweiten Spritze, der an einem proximalen Abschnitt davon angeordnet ist, zusammenpasst.

14. Fluidabgabesystem nach einem der vorhergehenden Ansprüche, wobei eine Querschnittsfläche des Zylinders 4 der ersten Spritze gleich einer Querschnittsfläche des Zylinders 9 der zweiten Spritze ist.

15. Fluidabgabesystem nach einem der vorhergehenden Ansprüche, wobei sich eine Querschnittsfläche eines Zylinders 4 der ersten Spritze von einer Querschnittsfläche des Zylinders 9 der zweiten Spritze unterscheidet.

16. Fluidabgabesystem nach einem der vorhergehenden Ansprüche, ferner umfassend:
c. eine Fluidaustragleitung 21, die zur Aufnahme von aus dem Zylinder 9 der zweiten Spritze ausgetragenen Fluids konfiguriert ist, wobei ein Auslass des Zylinders 4 der ersten Spritze in Fluidverbindung mit einer Austrittsstelle der Fluidaustragleitung 21 steht, so dass sich über die Fluidaustragleitung 21 aus dem Zylinder 9 der zweiten Spritze austretende Fluide mit aus dem Zylinder 4 der ersten Spritze austretenden Fluiden vermischen, und wahlweise ferner umfassend ein Rückschlagventil 20, das zur Regulierung des Durchflusses durch die Fluidaustragleitung 21 konfiguriert ist, um von Fluiden aus dem Auslass davon zurück in den Zylinder 9 der zweiten Spritze im Wesentlichen zu verhindern.

17. Fluidabgabesystem nach Anspruch 16, ferner umfassend einen Fluidabgabekatheter 19, der mindestens ein darin angeordnetes Lumen umfasst, das dahingehend betätigbar ist, das Gemisch von Fluiden von den Zylindern 4, 9 der ersten und der zweiten Spritze aufzunehmen.

18. Fluidabgabevorrichtung nach einem der vorhergehenden Ansprüche, ferner umfassend:
einen Dornbecher 14, der eine Dornbecherleitung darin zwischen einem Boden des Dornbechers und einer inneren Stelle im Dornbecher aufweist, wobei ein oberes Ende der Leitung ein scharfes Ende zum Durchstechen eines Septums eines Fläschchen-Reservoirs 16 ist, das ein ladbares Fluid enthält, einen Ladeanschluss 15, der direkt oder indirekt an einem gegebenen Zylinder einer der Spritzen angebracht und dazu konfiguriert ist, ein unteres Ende der Dornbecherleitung aufzunehmen, so dass, wenn Eingriff hergestellt ist, Fluid durch die Dornbecherleitung in den gegebenen Zylinder fließt, um den gegebenen Zylinder zu laden, wahlweise wobei der Ladeanschluss zu einer offenen und einer geschlossenen Konfiguration drehbar ist, so dass der Ladeanschluss nur dann zur Aufnahme von Fluid darinnen offen ist, wenn er in der offenen Konfiguration ist.

19. Fibrinogen und Thrombin zur Verwendung in einem Therapieverfahren, wobei das Fibrinogen und das Thrombin mittels eines Verfahrens zur Abgabe von Fluiden verabreicht werden, das Folgendes umfasst:
Bereitstellen einer Spritzenanordnung, die Folgendes umfasst:
i. eine erste und eine zweite Spritze, die gleich ausgerichtet sind, um eine Verabreichungsrichtung zu definieren, wobei jede Spritze einen jeweiligen Kolben 6, 10 hat, der in einem jeweiligen Spritzenzylinder 4, 9 verschiebbar ist, wobei der Kolben 10 der zweiten Spritze eine verstellbare Länge hat, und
ii. ein Koppelelement 8, das an dem Kolben 6 der ersten Spritze angebracht ist und damit mechanisch in Eingriff steht,
Verlängern oder Verkürzen des Kolbens 10 mit verstellbarer Länge der zweiten Spritze, um zu veranlassen, dass der Kolben 10 mit verstellbarer Länge der zweiten Spritze das Koppelelement 8 mechanisch in Eingriff nimmt bzw. daraus ausrückt, und
Bewegen des Koppelelements 8 in der Verabreichungsrichtung, um zu veranlassen, dass jeder in Eingriff stehende Kolben in Längsrichtung in seinem jeweiligen Spritzenzylinder 4, 9 gleitet, um sich übereinstimmend mit dem Koppelelement 8 zu bewegen und dadurch zu veranlassen, dass Fibrinogen und Thrombin enthaltende Fluide gleichzeitig aus jeweiligen Zylindern ausgestoßen werden.

## Revendications

1. Système d'administration de fluides, le système comprenant :
a. un ensemble seringues comprenant une première et une deuxième seringue de même orientation qui définissent une direction de préparation, chaque seringue comportant son piston (6, 10) respectif pouvant coulisser à l'intérieur d'un cylindre (4, 9) de seringue correspondant,
**caractérisé en ce que** le piston (10) de la deuxième seringue a une longueur réglable ; et
**caractérisé en outre en ce que** le système comprend :
b. un élément d'accouplement (8) fixé au piston (6) de la première seringue et en prise mécanique avec celui-ci de façon à ce que :
i. allonger ou raccourcir le piston (10) de longueur réglable de la deuxième seringue entraîne respectivement l'entrée en prise mécanique du piston (10) de longueur réglable de la deuxième seringue avec l'élément d'accouplement (8) ou sa désolidarisation de celui-ci ; et
ii. le mouvement de l'élément d'accouplement (8) dans la direction de préparation fait coulisser longitudinalement chaque piston en prise mécanique à l'intérieur de son cylindre (4, 9) de seringue correspondant dans un mouvement à l'unisson de l'élément d'accouplement (8).

2. Système d'administration de fluides selon la revendication 1, dans lequel l'élément d'accouplement (8) est fixé en permanence au piston (6) de la première seringue ; ou dans lequel l'élément d'accouplement (8) fait partie intégrante du piston (6) de la première seringue et/ou est collé à celui-ci ; ou dans lequel l'élément d'accouplement (8) est bloqué sur le piston (6) de la première seringue ; ou dans lequel l'élément d'accouplement (8) est fixé amovible au piston (6) de la première seringue ; ou dans lequel l'élément d'accouplement (8) est claveté sur le piston (6) de la première seringue.

3. Système d'administration de fluides selon la revendication 1 ou 2, dans lequel :
i. la direction de préparation définie par les première et deuxième seringues et
ii. la direction principale de contact entre les surfaces de contact (86, 84) respectives de l'élément d'accouplement (8) et du piston (10) de longueur réglable de la deuxième seringue, quand ils sont en prise mécanique l'un avec l'autre
ont la même orientation.

4. Système d'administration de fluides selon l'une quelconque des revendications précédentes, dans lequel le piston (10) de la deuxième seringue comprend un mécanisme à vis configuré pour modifier la longueur du piston (10) de la deuxième seringue, éventuellement dans lequel le piston (10) de la deuxième seringue comprend :
i. un manchon (74) à filetage interne ; et
ii. une tige (72) à filetage externe disposée à l'intérieur du manchon à filetage interne de telle sorte que la rotation de la tige à l'intérieur du manchon (74) provoque un déplacement longitudinal de la tige par rapport au manchon, réglant de ce fait la longueur du piston (10) de la deuxième seringue.

5. Système d'administration de fluides selon l'une quelconque des revendications précédentes, dans lequel la rotation d'un élément rotatif autour d'un axe parallèle à l'axe central de la deuxième seringue et/ou colinéaire avec celui-ci est destinée à régler la longueur du piston (10) de longueur réglable de la deuxième seringue pour qu'il entre en prise avec l'élément d'accouplement (8) ou s'en désolidarise.

6. Système d'administration de fluides selon l'une quelconque des revendications précédentes, dans lequel l'ensemble seringues comprend en outre une troisième seringue qui a la même orientation que les première et deuxième seringues, le piston (7) de la troisième seringue étant accouplé mécaniquement à l'élément d'accouplement (8) de telle sorte que le mouvement de celui-ci fait coulisser longitudinalement le piston (7) de la troisième seringue à l'intérieur de son cylindre (5) de seringue correspondant dans un mouvement à l'unisson de l'élément d'accouplement (8).

7. Système d'administration de fluides selon la revendication 6, dans lequel l'élément d'accouplement (8) est fixé en permanence au piston (7) de la troisième seringue ; ou dans lequel l'élément d'accouplement (8) fait partie intégrante du piston (7) de la troisième seringue et/ou est collé à celui-ci ; ou dans lequel l'élément d'accouplement (8) est bloqué sur le piston (7) de la troisième seringue ; ou dans lequel l'élément d'accouplement (8) est fixé amovible au piston (7) de la troisième seringue ; ou dans lequel l'élément d'accouplement (8) est claveté sur le piston (7) de la troisième seringue.

8. Système d'administration de fluides selon la revendication 6 ou 7, dans lequel l'axe central (60) de la deuxième seringue est sensiblement équidistant des axes centraux de la première et de la troisième seringue.

9. Système d'administration de fluides selon l'une quelconque des revendications 6 à 8, dans lequel la section transversale du cylindre (4) de la première seringue et/ou la section transversale du cylindre (9) de la deuxième seringue sont égales à la section transversale du cylindre (5) de la troisième seringue.

10. Système d'administration de fluides selon l'une quelconque des revendications 6 à 8, dans lequel la section transversale du cylindre (4) de la première seringue et/ou la section transversale du cylindre (9) de la deuxième seringue sont différentes de la section transversale du cylindre (5) de la troisième seringue.

11. Système d'administration de fluides selon l'une quelconque des revendications 6 à 10, comprenant en outre un cathéter (19) d'administration de fluides comprenant une première et une seconde lumière qui y sont incorporées en couvrant pratiquement la totalité de celui-ci et sont configurées pour recevoir respectivement les composants fluides déversés depuis les cylindres (4, 5) respectifs des première et troisième seringues afin de définir des canaux séparés de refoulement de composants fluides au bout (82) du cathéter (19).

12. Système d'administration de fluides selon l'une quelconque des revendications précédentes, dans lequel le rapport entre les longueurs plus longue et plus courte du piston (10) de la deuxième seringue est d'au moins 1,2 ; et/ou dans lequel la différence de longueur entre les longueurs plus longue et plus courte du piston (10) de la deuxième seringue est d'au moins 1 cm ; et/ou dans lequel la différence entre les longueurs plus longue et plus courte du piston (10) de la deuxième seringue est d'au moins 15% de la longueur intérieure du cylindre de la deuxième seringue.

13. Système d'administration de fluides selon l'une quelconque des revendications précédentes, dans lequel l'élément d'accouplement (8) comporte un évidement (44) dimensionné pour aller avec une partie en saillie (42) du piston (10) de la deuxième seringue, située au niveau de la partie proximale de celui-ci.

14. Système d'administration de fluides selon l'une quelconque des revendications précédentes, dans lequel la section transversale du cylindre (4) de la première seringue est égale à la section transversale du cylindre (9) de la deuxième seringue.

15. Système d'administration de fluides selon l'une quelconque des revendications précédentes, dans lequel la section transversale du cylindre (4) de la première seringue est différente de la section transversale du cylindre (9) de la deuxième seringue.

16. Système d'administration de fluides selon l'une quelconque des revendications précédentes, comprenant en outre :
c. une conduite (21) de décharge de fluides configurée pour recevoir les fluides déversés depuis le cylindre (9) de la deuxième seringue, une sortie du cylindre (4) de la première seringue étant en communication fluidique avec un endroit de sortie de la conduite (21) de décharge de fluides de façon à ce que les fluides sortant du cylindre (9) de la deuxième seringue via la conduite (21) de décharge de fluides se mélangent avec les fluides sortant du cylindre (4) de la première seringue ; et éventuellement comprenant en outre un clapet antiretour (20) configuré pour réguler l'écoulement par la conduite (21) de décharge de fluides afin d'empêcher pratiquement de fluides venant de sa sortie en retour dans le cylindre (9) de la deuxième seringue.

17. Système d'administration de fluides selon la revendication 16, comprenant en outre un cathéter (19) d'administration de fluides comprenant au moins une lumière qui y est située et sert à recevoir le mélange de fluides provenant des cylindres (4, 9) des première et deuxième seringues.

18. Dispositif d'administration de fluides selon l'une quelconque des revendications précédentes, comprenant en outre :
une cuvette (14) à pointe comprenant à l'intérieur une conduite de cuvette à pointe entre le fond de la cuvette à pointe et un endroit intérieur à l'intérieur de la cuvette à pointe, l'extrémité supérieure de la conduite étant une extrémité aiguë pour percer le septum d'un réservoir (16) en forme de fiole contenant un fluide chargeable ;
un orifice de chargement (15) directement ou indirectement fixé à un cylindre donné d'une des seringues et configuré pour recevoir l'extrémité inférieure de la cuvette à pointe afin que quand ils sont en prise le fluide de celle-là passe par la conduite de cuvette à pointe et entre dans le cylindre donné de façon à charger le cylindre donné ; éventuellement dans lequel l'orifice de chargement peut tourner pour adopter des configurations d'ouverture et de fermeture de telle sorte que c'est seulement quand il est en configuration ouverte que l'orifice de chargement est ouvert pour recevoir du fluide.

19. Fibrinogène et thrombine à utiliser dans un procédé thérapeutique dans lequel lesdits fibrinogène et thrombine sont administrés par un procédé d'administration de fluides comprenant les opérations consistant à :
faire appel à un ensemble seringues comprenant :
i. une première et une deuxième seringue de même orientation qui définissent une direction de préparation, chaque seringue comportant son piston (6, 10) respectif pouvant coulisser à l'intérieur d'un cylindre (4, 9) de seringue correspondant, le piston (10) de la deuxième seringue ayant une longueur réglable ; et
ii. un élément d'accouplement (8) fixé au piston (6) de la première seringue et en prise mécanique avec celui-ci ;
allonger ou raccourcir le piston (10) de longueur réglable de la deuxième seringue de façon à entraîner l'entrée en prise mécanique du piston (10) de longueur réglable de la deuxième seringue avec l'élément d'accouplement (8) ou sa désolidarisation de celui-ci ; et
déplacer l'élément d'accouplement (8) dans la direction de préparation de façon à faire coulisser longitudinalement chaque piston en prise à l'intérieur de son cylindre (4, 9) de seringue correspondant dans un mouvement à l'unisson de l'élément d'accouplement (8) et de ce fait provoquer l'expulsion simultanée depuis les cylindres respectifs des fluides comprenant le fibrinogène et la thrombine.
